Europäisches Patentamt

(19) European Patent Office

Office européen des brevets

(11) Numéro de publication : **0 128 818**
**B1**

(12) **FASCICULE DE BREVET EUROPÉEN**

(45) Date de publication du fascicule du brevet :
**30.12.86**

(21) Numéro de dépôt : **84401148.6**

(22) Date de dépôt : **05.06.84**

(51) Int. Cl.⁴ : **C 07 C 19/02**, C 07 C 19/04,
C 07 C 19/06, C 07 C 17/10

(54) **Procédé de fabrication de chlorométhanes supérieurs.**

(30) Priorité : **10.06.83 FR 8309667**

(43) Date de publication de la demande :
**19.12.84 Bulletin 84/51**

(45) Mention de la délivrance du brevet :
**30.12.86 Bulletin 86/52**

(84) Etats contractants désignés :
**AT BE DE FR GB IT NL SE**

(56) Documents cités :
**FR-A- 2 081 871**
**PATENTS ABSTRACTS OF JAPAN, volume 4, no. 77
(C-13)(559), 4 juin 1980, page 91C13**

(73) Titulaire : **ATOCHEM**
**4 & 8, Cours Michelet La Défense 10**
**F-92800 Puteaux (FR)**

(72) Inventeur : **Masini, Jean-Jacques**
**35, rue Jean Penet**
**F-69639 Chaponost (FR)**
Inventeur : **Verot, Yvan**
**"Le Prieuré" 20 Rue du Prieuré**
**F-69130 Ecully (FR)**

(74) Mandataire : **Rochet, Michel et al**
**ATOCHEM Département Propriété Industrielle**
**F-92091 Paris la Défense Cédex 42 (FR)**

## Description

La présente invention concerne un procédé de fabrication de chlorométhanes supérieurs par chloration du chlorure de méthyle en phase liquide de type radicalaire avec initiation chimique ou photochimique, permettant la production simultanée de quantités choisies des trois chlorométhanes supérieurs $CH_2Cl_2$, $CHCl_3$ et $CCl_4$.

Les procédés classiques de fabrication de chlorométhanes supérieurs par chloration de $CH_3Cl$ ne concernent pratiquement que la production de $CH_2Cl_2$ et $CHCl_3$. Le $CCl_4$ co-produit représente moins de 15 % de la production totale et est considéré comme un sous-produit de la réaction.

La production de $CCl_4$ est généralement assurée par des procédés dits « Tetra-per » (tetrachlorure de carbone-perchloréthylène) à partir de résidus chlorés et/ou d'hydrocarbures saturés ou oléfiniques à deux et trois atomes de carbone. De telles techniques conduisent cependant à générer des sous-produits lourds en quantité non négligeable. Elles sont en outre fortement consommatrices d'énergie.

A cause de ces inconvénients des procédés classiques, la fabrication du $CCl_4$ à partir des chlorométhanes provenant de la chloration du $CH_3Cl$ pourrait être intéressante, surtout que les plus récents perfectionnements de ce procédé permettent la chloration de type radicalaire en phase liquide à basse température, avec initiateur chimique ou photochimique. De tels procédés sont économiques du fait des rendements élevés obtenus et de la simplification des séparations, peu de sous-produits gênants étant fabriqués.

Cependant dans l'état actuel de la technique, la production simultanée de quantités imposées et éventuellement voisines des trois chlorométhanes supérieurs ne va pas sans poser de problèmes dans une unité classique constituée d'un réacteur de chloration du $CH_3Cl$ suivi de colonnes épuratrices et séparatrices des différents produits fabriqués. Un tel système manque de flexibilité. En effet, dans ce type d'unité, la principale contrainte se trouve être l'ajustement des productions respectives de $CH_2Cl_2$ et $CHCl_3$ ; la quantité de $CCl_4$ produite simultanément est fonction de la proportion $CH_2Cl_2$—$CHCl_3$ effectivement obtenue et des conditions opératoires. Cette production de $CCl_4$ ne peut être que faible par rapport à celle des deux autres chlorométhanes.

Vouloir dans une unité classique dotée d'un seul réacteur de chloration produire des quantités imposées et éventuellement voisines des trois chlorométhanes supérieurs rejaillit fortement sur le montant des investissements auxquels il faut consentir. En effet dans ce type d'unité l'ajustement de la flexibilité est obtenu par la limitation du taux de transformation de $CH_3Cl$ et éventuellement par un recyclage approprié de $CH_2Cl_2$. Ceci se traduit déjà par un surdimensionnement important des ensembles d'appareils concernés entraînant une augmentation des frais de fonctionnement. Par ailleurs la productivité d'un réacteur donné, toute chose égale par ailleurs est sensible à la composition de la production en chlorométhanes supérieurs. Il est connu que la vitesse de chloration d'un chlorométhane diminue sensiblement quand augmente le nombre d'atomes de chlore contenus dans la molécule : un réacteur ayant une productivité de 100 pour des compositions respectives en $CH_2Cl_2$, $CHCl_3$ et $CCl_4$ voisines de 45/45/10 % poids, n'aura plus, toute chose égale par ailleurs, qu'une productivité voisine de 65 pour une répartition de 37,5/47,5/15 et de 30 pour 20/45/35. La productivité est définie par la quantité de chlore réagie par unité de volume de réacteur et ce pour un taux fixé de chlore non réagi en sortie du réacteur.

Le procédé selon l'invention consiste à faire fonctionner en parallèle deux réacteurs A et B de chloration en phase liquide de type radicalaire avec initiation chimique ou photochimique, le réacteur A fonctionnant de manière classique en chloration de chlorure de méthyle, le réacteur B alimenté en chlore et en $CH_2Cl_2$ et/ou $CHCl_3$ fabriqués dans le réacteur A servant à produire soit simultanément des chlorométhanes supérieurs $CHCl_3$ et $CCl_4$ soit $CCl_4$ seul et à assurer une grande flexibilité à l'ensemble de l'unité de fabrication.

Les températures dans les réacteurs sont comprises entre 50 et 120 °C et de préférence 60 et 90 °C avec des pressions de 10 à 50 bars et de préférence de 15 à 25 bars. Le rapport dans le réacteur A entre le nombre de molécules de $Cl_2$ et le nombre de molécules de $CH_3Cl$ est compris entre 0,3 et 1,5 et de préférence 0,5 et 1. Les quantités de $Cl_2$ introduites dans le réacteur B sont adaptées en fonction des quantités de chacun des chlorométhanes supérieurs introduits et du ou des chlorométhanes supérieurs que l'on souhaite finalement obtenir. Les réactions de chloration dans les réacteurs A et B sont connues en elles-mêmes.

Selon le procédé l'ajustement des productions de $CH_2Cl_2$, $CHCl_3$, $CCl_4$ se fait en adaptant d'une part la répartition et la quantité des produits formés dans le réacteur A et d'autre part la production et le ratio des quantités transformées de $CH_2Cl_2$ et de $CHCl_3$ du réacteur B.

Le schéma de principe en annexe permet de mieux comprendre le fonctionnement du procédé. Par les tubulures 1 et 2 on alimente le réacteur A respectivement en $Cl_2$ et en $CH_3Cl$. Les produits de réaction sont envoyés par la tubulure 3 dans un ensemble de séparation D : HCl—$CH_3Cl$-chlorométhanes supérieurs, HCl étant évacué par la tubulure 4 et $CH_3Cl$ et les chlorométhanes supérieurs étant envoyés par la tubulure 5 dans l'ensemble de séparation E. $CH_3Cl$ non réagi est évacué par la tubulure 8 ou encore recyclé par la tubulure 6 au réacteur A. Afin de limiter la température du pied de cuve de l'ensemble D il est également possible de recycler plus ou moins de $CH_3Cl$ par la tubulure 7. Les chlorométhanes supérieurs sont envoyés par la tubulure 9 dans l'ensemble de séparation F du $CH_2Cl_2$ des autres chlorométhanes supérieurs, le $CH_2Cl_2$ pouvant être récupéré en tout ou partie en 12 ou recyclé en tout ou partie par la tubulure 13 dans le réacteur B. Enfin les chlorométhanes supérieurs restant sont envoyés par la tubulure 11 dans l'ensemble de séparation G du $CHCl_3$ du $CCl_4$ brut, le $CHCl_3$ pouvant être récupéré en tout ou partie en 16 ou recyclé en tout ou partie par la tubulure 17 dans le réacteur B, le $CCl_4$ brut étant récupéré en 15. Il peut être envisagé de recycler des chlorométhanes supérieurs dans le réacteur B par les tubulures 10 et 14. Dans ce cas, du $CCl_4$ n'interférant pas sur les produits de la réaction se trouve recyclé dans le réacteur B. Ce $CCl_4$ joue alors le rôle de diluant des réactifs et par là peut ralentir la réaction ; en conséquence cette recirculation doit de préférence être limitée.

Avant d'introduire les chlorométhanes supérieurs dans le réacteur B, il peut être intéressant de les faire circuler préalablement dans un dispositif C de lavage des gaz permettant de laver la phase gazeuse issue du réacteur B.

Les chlorométhanes supérieurs réagissent dans le réacteur B avec du $Cl_2$ introduit par la tubulure 18. Les produits de réaction sont regroupés par la tubulure 19 à ceux du réacteur A de façon la plus simple dans l'ensemble de séparation D.

Il est recommandé, bien que cela ne soit pas indispensable que les chlorométhanes alimentant le réacteur B contiennent le moins possible de $CH_3Cl$ pour que ce réacteur conserve à la fois une sélectivité et une productivité acceptables pour des taux de transformation donnés du $CH_2Cl_2$ et ou du $CHCl_3$.

L'ajustement des quantités de $CH_2Cl_2$ transformées et de $CHCl_3$ transformées ou produites se fait par adaptation de la composition en $CH_2Cl_2$ et $CHCl_3$ dans le réacteur ainsi que du débit de son alimentation. Les paramètres réactionnels de chacun des réacteurs, et bien entendu les réacteurs eux-mêmes sont adaptés de manière à ce que les réactions aient lieu en phase liquide. Les pressions dans chaque réacteur sont généralement voisines et doivent être choisies de façon à ce que la séparation HCl-chlorométhanes soit facilitée.

Les produits de réaction quittent les réacteurs A et B sous forme liquide ou gazeuse. Il est possible, quoique cela ne soit pas indispensable, d'effectuer le lavage des phases gazeuses par du chlorométhane supérieur afin de récupérer les traces de chlore qu'elles contiennent et qui représentent la plus grosse partie du chlore non transformé. Ce lavage est particulièrement intéressant pour les produits du réacteur B. Un tel lavage permet de récupérer l'excès de chlore et par là d'augmenter significativement la productivité du réacteur.

Les différents produits de réaction des réacteurs A et B sont séparés les uns des autres dans les ensembles de séparation présentées précédemment, dans les conditions habituelles de distillation propres à chacun de ces produits.

Le procédé de l'invention présente l'avantage de concentrer les problèmes de limitation de productivité sur le seul réacteur B auxiliaire, ce qui les atténue très sensiblement par le fait que la matière réactionnelle de ce réacteur est formée de $CH_2Cl_2$ et/ou de $CHCl_3$. Un autre avantage ressort de la flexibilité particulièrement importante de l'ensemble réactionnel qui conserve par ailleurs tous les avantages économiques du procédé classique. En effet, pour une production de $CCl_4$ donnée pouvant aller jusqu'à 50 %· de la production totale, la répartition $CH_2Cl_2$—$CHCl_3$ du complément de production peut varier continûment dans une large proportion et plus particulièrement du point de vue économique entre 75/25 et 25/75 % poids.

Les exemples suivants illustrent l'invention sans toutefois la limiter.

Exemple 1

Un réacteur A est alimenté en continu par 0,15 kmoles/h de chlore liquide et 0,185 kmoles/h de $CH_3Cl$ liquide. Ce réacteur est un réacteur photochimique parfaitement agité, travaillant sous 15 bars absolus et à 60 °C refroidi par une circulation d'eau ; les produits de réaction ont la composition approximative suivants (mélange liquide + vapeur) :

% poids
HCl   = 27,2
$CH_3Cl$ = 22,7
$Cl_2$   < 0,5 % du mélange des autres composés
$CH_2Cl_2$ = 22,1
$CHCl_3$  = 22
$CCl_4$   = 6

Un réacteur B est alimenté en continu par 0,021 kmoles/h de chlore liquide et 0,043 kmoles/h de $CHCl_3$ liquide. Ce réacteur est identique au réacteur A, et travaille avec les·mêmes paramètres de fonctionnement. Les produits de réaction ont la composition approximative suivante (mélange liquide + vapeur) :

% poids
HCl = 11,8

3

$Cl_2 < 1$ % du mélange HCl + $CHCl_3$ + $CCl_4$
$CHCl_3 = 38,5$
$CCl_4 = 49,7$

Les deux mélanges réactionnels alimentent une colonne de distillation travaillant sous 12 bars absolus, dans laquelle l'HCl est séparé des autres produits réactionnels. On obtient un soutirage ayant la composition approximative suivante :

% poids
$CH_3Cl = 22,2$
$CH_2Cl_2 = 21,7$
$CHCl_3 = 34,1$
$CCl_4 = 22$

Ce mélange ainsi obtenu est ensuite séparé par distillation successive, de manière à obtenir chacun des chlorométhanes. On obtient ainsi l'équivalent de :

k moles/h
$CH_3Cl = 0,089$
$CH_2Cl_2 = 0,051$
$CHCl_3 = 0,058$
$CCl_4 = 0,029$

Si on tient compte alors du recyclage des 0,043 kmoles/h de $CHCl_3$ recyclées vers le réacteur B, la production de chlorométhanes supérieurs est alors voisine de :

k moles/h
$CH_2Cl_2 = 0,051$
$CHCl_3 = 0,015$
$CCl_4 = 0,029$

ce qui conduit à une proportion pondérale du mélange $CH_2Cl_2$ + $CHCl_3$ en $CH_2Cl_2$ et $CHCl_3$ respectivement de 70,3 % et 29,7 %.

Exemple 2

L'essai est repris dans les conditions de l'exemple 1, mais en ajoutant au chloroforme alimenté au réacteur B un appoint de $CCl_4$ correspondant à environ 20 % poids du chloroforme. La seule modification notable par rapport aux résultats obtenus dans l'exemple 1 est l'augmentation du chlore non transformé dans le réacteur B.

Exemple 3

Le réacteur A fonctionne dans les mêmes conditions que celles décrites dans l'exemple 1.
Le réacteur B est cette fois alimenté par 0,029 kmoles/h de $CH_2Cl_2$ liquide et 0,05 kmoles/h de chlore liquide. Il travaille avec les mêmes paramètres de fonctionnement que précédemment. Les produits de réaction ont la composition approximative suivante (mélange liquide + vapeur) :

% poids
$HCl = 30$
$Cl_2 < 95$ du mélange total
$CH_2Cl_2 = 2$
$CHCl_3 = 14$
$CCl_4 = 54$

Après séparation des produits, on recueille alors l'équivalent de :

k moles/h
$CH_2Cl_2 = 0,053$
$CHCl_3 = 0,043$
$CCl_4 = 0,029$

Si on tient compte du recyclage des 0,029 kmoles/h de $CH_2Cl_2$ vers le réacteur B, la production de chlorométhanes supérieurs est alors voisine de :

k moles/h
$CH_2Cl_2 = 0,024$
$CHCl_3 = 0,043$
$CCl_4 = 0,029$

ce qui conduit à une proportion pondérale du mélange $CH_2Cl_2 + CHCl_3$ en $CH_2Cl_2$ et $CHCl_3$ respectivement de 27,8 % et 72,2 %.

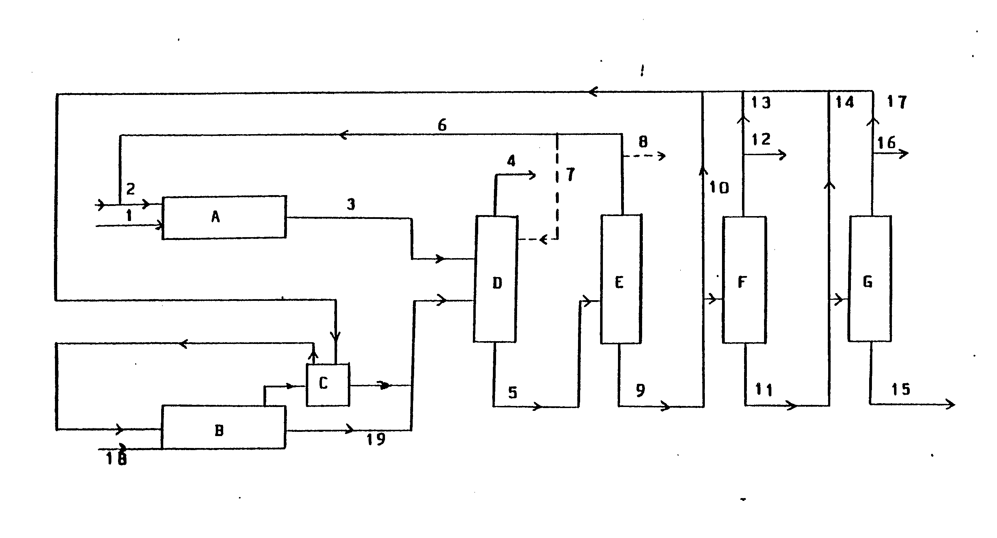

## Revendications

1. Procédé de fabrication permettant la fabrication simultanée de quantités choisies de $CH_2Cl_2$, $CHCl_3$ et $CCl_4$ par chloration en phase liquide de type radicalaire avec initiation chimique ou photochimique de $CH_3Cl$ caractérisé en ce que :

a. Dans un réacteur B on chlore du $CH_2Cl_2$ et/ou $CHCl_3$ fabriqué dans un réacteur en parallèle A de chloration du $CH_3Cl$.

b. Les températures dans les deux réacteurs sont comprises entre 50 et 120 °C pour des pressions comprises entre 10 et 50 bars.

c. Dans le réacteur A le rapport entre le nombre de molécules de $Cl_2$ et le nombre de molécules de $CH_3Cl$ est compris entre 0,3 et 1,5.

d. Les quantités de $Cl_2$ introduites dans le réacteur B sont adaptées en fonction des quantités de chacun des chlorométhanes supérieurs introduits et du ou des chlorométhanes supérieurs que l'on souhaite finalement obtenir.

2. Procédé selon la revendication 1 caractérisé en ce que le $CH_2Cl_2$ et/ou $CHCl_3$ est dilué par du $CCl_4$.

3. Procédé selon l'une des revendications 1 à 2 caractérisé en ce que la phase gazeuse provenant du réacteur B est lavée par du chlorométhane supérieur provenant du réacteur A.

4. Procédé selon l'une des revendications 1 à 3 caractérisé en ce que les produits formés dans les réacteurs A et B sont réunis avant la séparation du ou des chlorométhanes recyclés à l'alimentation du réacteur B.

## Claims

1. Manufacturing process which permits simultaneous manufacture of chosen amounts of $CH_2Cl_2$, $CHCl_3$ and $CCl_4$ by radical-type liquid phase chlorination of $CH_3Cl$, with chemical or photochemical initiation, characterized in that :

a. $CH_2I_2$ and/or $CHCl_3$ which have been manufactured in a parallel reactor A for the chlorination of $CH_3Cl$ are chlorinated in a reactor B,

b. the temperatures in the two reactors are between 50 and 120 °C, for pressures of between 10 and 50 bars,

c. in the reactor A, the molar ratio of the number of molecules of $Cl_2$ to the number of molecules of $CH_3Cl$ is between 0.3 and 1.5,

d. the amounts of $Cl_2$ introduced into the reactor B are adapted in accordance with the amounts of each of the higher chloromethanes introduced and of the higher chloromethane or choromethanes which it is desired to obtain finally.

2. Process according to Claim 1, characterized in that the $CH_2Cl_2$ and/or $CHCl_3$ is diluted with $CCl_4$.

3. Process according to one of Claims 1 and 2, characterized in that the gas phase coming from the reactor B is washed with some of the higher chloromethane coming from the reactor A.

4. Process according to one of Claims 1 to 3, characterized in that the products from the reactors A and B are combined before separation of the chloromethane or choromethanes recycled to the feed of the reactor B.

## Patentansprüche

1. Verfahren zur gleichzeitigen Herstellung vorgegebener Mengen von $CH_2Cl_2$, $CHCl_3$ und $CCl_4$ durch radikalische Chlorierung in der Flüssigphase von $CH_3Cl$ mit chemischen oder photochemischen Kettenstart, gekennzeichnet durch die nachfolgend genannten Merkmale :

a. in einem Reaktor B werden $CH_2Cl_2$ und/oder $CHCl_3$ chloriert, die in einem zweiten parallelen Reaktor A durch Chlorierung von $CH_3Cl$ gewonnen wurden ;

b. die Temperatur in beiden Reaktoren wird zwischen 50 und 120 °C gehalten und der Druck in beiden Reaktoren wird zwischen 10 und 50 bar gehalten ;

c. im Reaktor A liegt das Verhältnis zwischen der Zahl der $Cl_2$-Moleküle und der Zahl der $CH_3Cl$ Moleküle zwischen 0,3 und 1,5 ;

5

d. die in den Reaktor B eingeführten $Cl_2$-Menge wird jeweils entsprechend der Menge des jeweils eingeleiteten höheren Chlormethans einerseits und der Menge des herzustellenden höheren Chlormethans gewählt.

2. Verfahren nach Anspruch 1, dadurch gekennzeichnet, daß $CH_2Cl_2$ und/oder $CHCl_3$ durch $CCl_4$ verdünnt werden.

3. Verfahren nach einem der Ansprüche 1 und 2, dadurch gekennzeichnet, daß die aus dem Reaktor B entnommene Gasphase mit dem aus dem Reaktor A entnommenen höheren Chlormethan gewaschen wird.

4. Verfahren nach einem der Ansprüche 1 bis 3, dadurch gekennzeichnet, daß die in den Reaktoren A und B gebildeten Produkte vor der Auftrennung in die Chlormethane vereinigt und in die Zuleitung des Reaktors B zurückgeführt werden.